(19) Europäisches Patentamt European Patent Office Office européen des brevets

(11) **EP 4 035 709 A1**

(12) **EUROPEAN PATENT APPLICATION**
published in accordance with Art. 153(4) EPC

(43) Date of publication:
**03.08.2022 Bulletin 2022/31**

(21) Application number: **19946744.0**

(22) Date of filing: **25.09.2019**

(51) International Patent Classification (IPC):
**A61M 5/20** (2006.01)    **A61M 5/142** (2006.01)

(52) Cooperative Patent Classification (CPC):
**A61M 5/142; A61M 5/20**

(86) International application number:
**PCT/CN2019/107793**

(87) International publication number:
**WO 2021/056249 (01.04.2021 Gazette 2021/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Shenzhen Mindray Scientific Co., Ltd.**
**Shenzhen, Guangdong 518000 (CN)**

(72) Inventors:
• YANG, Lei
  **Shenzhen, Guangdong 518000 (CN)**
• ZHANG, Peng
  **Shenzhen, Guangdong 518000 (CN)**
• ZOU, Xiaoling
  **Shenzhen, Guangdong 518000 (CN)**

(74) Representative: **KIPA AB**
**Drottninggatan 11**
**252 21 Helsingborg (SE)**

(54) **SYRINGE PUMP, METHOD FOR CONFIRMING INFUSION INFORMATION, MEDICAL DEVICE AND STORAGE MEDIUM**

(57)    A syringe pump (10), comprising a syringe pump drive mechanism (20), a syringe clamping mechanism (21), a syringe propulsion mechanism (22), a processor (12), an output interface (13) and a display screen (14). The syringe clamping mechanism (21) is used for clamping a syringe body (23), and the syringe pump drive mechanism (20) is used for driving the syringe propulsion mechanism (22) to push a piston (24) of the syringe, so that the piston (24) and the syringe body (23) move relative to one another; and the processor (12) is used to connect to the display screen (14) by means of the output interface (13). After the syringe (11) is clamped in the syringe clamping mechanism (21) and a user inputs syringe model information, dose information is displayed on the display screen (14); and the dose information changes along with the operation of the syringe pump drive mechanism (20). Also disclosed are a method for confirming infusion information, a medical device and a storage medium. Thus, the dose information and/or injection time information may be accurately displayed, thereby improving injection accuracy.

FIG.3

EP 4 035 709 A1

**Description**

TECHNICAL FIELD

The disclosure relates to the technical field of medical devices, and more particularly to a syringe pump, a method for confirming infusion information, a medical device and a storage medium.

BACKGROUND

The syringe pump injects medicine solution in the syringe into the body of a patient through pushing a pushing handle. The infusion accuracy for the patient would be affected by the accurate calculation of the amount of medicine solution in the syringe and the accurate detection of the evacuation position of the syringe. In serious cases, it may lead to overflow or underflow conditions.

The prior syringe pump usually adopts the following scheme for infusion. Specifically, the medical staff estimates the amount of medicine and input the amount parameter into the syringe pump. The syringe pump implements infusion according to this parameter and gives the injection time. However, this method relies on the experience of the medical staff. When the amount parameter inputted is too less than the actual amount, the infusion pump may complete the infusion in advance which may delay the treatment for the patient. When the amount parameter inputted is too large than the actual amount, as the syringe pump cannot accurately acquire the evacuation position, the syringe pump cannot send the evacuation alarm in time, which may result in the occurrence of underflow and also delay the treatment for the patient.

SUMMARY

Based on these, a syringe pump, a method for confirming infusion information, a medical device and a storage medium, which are capable of accurately calculating a remaining amount and/or a remaining injection time of medicine, should be provided, aiming at the above-mentioned problems.

A syringe pump is provided, which includes a syringe pump drive mechanism, a syringe clamping mechanism, a syringe propulsion mechanism, a processor, an output interface and a display screen; wherein the syringe clamping mechanism is operable to clamp a body of a syringe, the syringe pump drive mechanism is operable to drive the syringe propulsion mechanism to push a piston of the syringe for enabling a relative movement between the body of the syringe and the piston of the syringe, the processor is operable to connect with the display screen through the output interface and is operable to display medicine amount information on the display screen after the syringe is clamped by the syringe clamping mechanism and syringe model information is inputted by a user; wherein the medicine amount information changes along with operation of the syringe pump drive mechanism.

A syringe pump is provided, which includes a syringe pump drive mechanism, a syringe clamping mechanism, a syringe propulsion mechanism, a processor, an output interface and a display screen; wherein the syringe clamping mechanism is operable to clamp a body of a syringe, the syringe pump drive mechanism is operable to drive the syringe propulsion mechanism to push a piston of the syringe for enabling a relative movement between the body of the syringe and the piston of the syringe, the processor is operable to connect with the display screen through the output interface and is operable to display injection time information on the display screen, after the syringe is clamped by the syringe clamping mechanism and syringe model information and a flow rate are inputted by a user; wherein the injection time information changes along with operation of the syringe pump drive mechanism.

A syringe pump is provided, which includes a syringe pump drive mechanism, a syringe clamping mechanism, a syringe propulsion mechanism, a processor, an output interface and a display screen; wherein the syringe clamping mechanism is operable to clamp a body of a syringe, the syringe pump drive mechanism is operable to drive the syringe propulsion mechanism to push a piston of the syringe for enabling a relative movement between the body of the syringe and the piston of the syringe, the processor is operable to connect with the display screen through the output interface and is operable to display medicine amount information and/or injection time information on the display screen after the syringe is clamped by the syringe clamping mechanism and syringe model information , as well as a flow rate, are inputted by a user; wherein the medicine amount information and/or the injection time information change(s) along with operation of the syringe pump drive mechanism; wherein the medicine amount information or the injection time information is displayed in a first display mode, wherein a ratio between the medicine amount information and a total infusion amount, or a contrast between the injection time information and a total infusion time, is displayed in a second display mode; wherein the first display mode or the second display mode includes text, number or graphics or combination thereof, and the first display mode is different from the second display mode.

A method for confirming infusion information of a syringe pump is provided, wherein the syringe pump includes a syringe pump drive mechanism, a syringe clamping mechanism, a syringe propulsion mechanism, a processor, an output interface and a display screen, and the method is applied to the syringe pump and includes:

clamping a syringe by the syringe clamping mechanism;

inputting syringe model information;
displaying medicine amount information;
wherein the medicine amount information changes along with operation of the syringe pump drive mechanism; and when the syringe pump drive mechanism operates, the syringe pump drive mechanism drives the syringe propulsion mechanism to push a piston of the syringe for enabling a relative movement between a body of the syringe and the piston of the syringe for implementing an operation.

A method for confirming infusion information of a syringe pump is provided, wherein the syringe pump includes a syringe pump drive mechanism, a syringe clamping mechanism, a syringe propulsion mechanism, a processor, an output interface and a display screen, and the method is applied to the syringe pump and includes:

clamping a syringe by the syringe clamping mechanism;
inputting syringe model information and a flow rate;
displaying injection time information;
wherein the injection time information changes along with operation of the syringe pump drive mechanism; and when the syringe pump drive mechanism operates, the syringe pump drive mechanism drives the syringe propulsion mechanism to push a piston of the syringe for enabling a relative movement between a body of the syringe and the piston of the syringe for implementing an operation.

A medical device is provided, which is operable to connect with the syringe pump described above through the output interface, wherein the medical device is provided with a display device which is operable to display instruction information which is outputted by the output interface.

A storage medium is provided, which stores executable instructions, wherein when the executable instructions are executed by a processor, the method above can be implemented.

The above syringe pump includes a syringe pump drive mechanism, a syringe clamping mechanism, a syringe propulsion mechanism, a processor, an output interface and a display screen. The syringe clamping mechanism is operable to clamp a body of a syringe, the syringe pump drive mechanism is operable to drive the syringe propulsion mechanism to push a piston of the syringe for enabling a relative movement between the body of the syringe and the piston, the processor is operable to connect with the display screen through the output interface and is operable to display medicine amount information on the display screen after the syringe is clamped by the syringe clamping mechanism and syringe model information is inputted by a user. Wherein the medicine amount information changes along with operation of the syringe pump drive mechanism. Among them, a displacement of the piston is acquired according to the relative movement between the piston and the body of the syringe. Then the medicine amount information can be determined according to the displacement of the piston and the syringe model information. In such a way, the medicine amount information can be accurately displayed to facilitate the user to carry out next operations.

BRIEF DESCRIPTION OF THE DRAWINGS

In order to explain embodiments of this disclosure more clearly, the following will briefly introduce drawings required in the description for the embodiments or the prior art description. It is obvious that the drawings in the following description are only some embodiments of this disclosure. For those skilled in the art, other drawings can be obtained from these accompanying drawings without paying any creative works.

FIG. 1 is a structural block diagram of a medical device in an embodiment of the present disclosure.
FIG. 2 is an application environment diagram of a syringe pump in an embodiment of the present disclosure.
FIG. 3 is a structural block diagram of a syringe pump in an embodiment of the present disclosure.
FIG. 4 is a structural block diagram of a syringe pump in an embodiment of the present disclosure.
FIG.5 is a structural block diagram of a syringe pump in an embodiment of the present disclosure.
FIG. 6 is a schematic diagram showing a syringe pump which clamps a syringe in an embodiment of the present disclosure.
FIG. 7 is a structural block diagram of a syringe pump in an embodiment of the present disclosure.
FIGS. 8A-8D are schematic diagrams of a display mode which is a progress bar for indication in an embodiment of the present disclosure.
FIGS. 9A-9B are schematic diagrams of sending out a reminder signal in an embodiment of the present disclosure.
FIG. 10 is a schematic diagram of a displaying in an embodiment of the present disclosure.

DESCRIPTION OF THE PREFERRED EMBODIMENTS

The technical solutions in example embodiments of the disclosure will be described clearly and completely below with reference to the accompanying drawings. Many specific details are shown in the following detailed description in order to provide a full understanding of the various described embodiments. However, it should be understood by those skilled in the art that the various described embodiments can be implemented without these specific details. In other embodiments, the known methods, processes, components, circuits and networks are not described in detail to avoid unnecessarily blurring the embodiments.

It will also be understood that although in some cases the terms, such as "first", "second", etc., are used herein to describe various elements or other objects, however, these elements or objects should not be limited by these terms. These terms are only used to distinguish one element/object from another.

The terms used in the description of various described embodiments herein are only for the purpose of describing specific embodiments and are not intended to give any limitations. As used in the description of the various described embodiments and the appended claims, the singular forms of "a" and "the/said" are intended to also include the plural form, unless the context expressly indicates otherwise. It will also be understood that the term "and/or" as used herein refers to and covers any and all possible combinations of one or more of the associated listed projects. It will also be understood that the terms "including", "comprising" and any variations thereof, when used in this specification, refers to the existence of the stated features, steps, operations, elements and/or components, but does not exclude the existence or addition of one or more other features, steps, operations, elements and/or components.

As used herein, depending on the context, the term "if' can be interpreted as meaning "when", "in response to determining" or "in response to detecting". Similarly, depending on the context, the phrase "if determined..." or "if [a stated condition or event] is detected" may be interpreted to mean "when determining...", "in response to determining...", "when [a stated condition or event] is detected" or "in response to a detection of [a stated condition or event]".

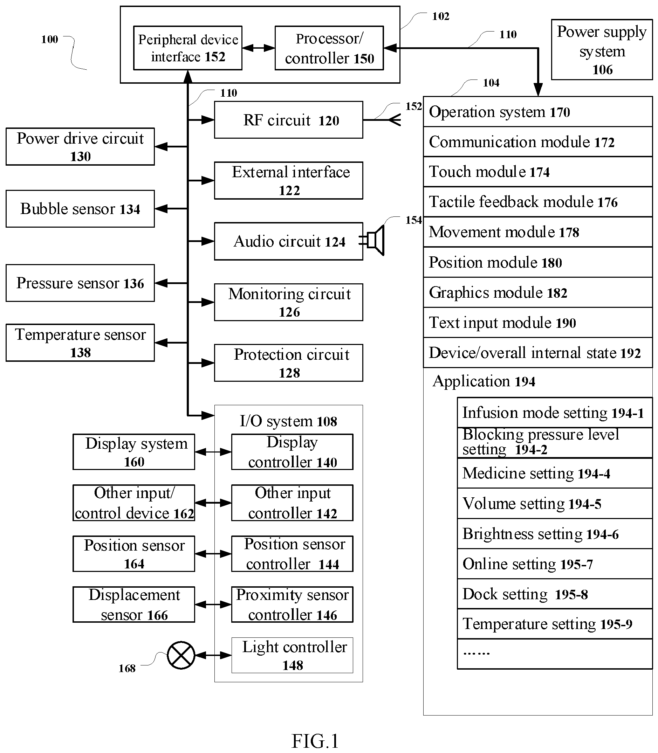

FIG. 1 illustrates a medical device according to some embodiments of the present disclosure. The medical device 10 includes a control platform 102, a memory 104, a power supply system 106, an input/output (I/O) system 108, an RF circuit 120, an external interface 122, an audio circuit 124, a monitoring circuit 126, a protection circuit 128, a power drive circuit 130, a drop sensor 132, a bubble sensor 134, a pressure sensor 136, a temperature sensor 138 and so on. These components communicate with each other through one or more communication buses or signal lines 101. The control platform 102 includes a processor/controller 150 and a peripheral device interface 152.

The medical device 10 may be any medical device that is capable of implementing infusion and injection operations which are preset by a user on liquid which is prepared by the user, for infusing the prepared medicine liquid into a patient controllably. The medical device includes but is not limited to an infusion pump, an analgesic pump, a nutrient pump, an insulin pump, and/or any combination thereof. In some embodiments, the medical device can be used together with the infusion apparatus (such as a syringe, an infusion tube). It should be understood that the medical device 10 is only an example, and the medical device may have more or fewer components than those are shown or have different component configurations. The various components described in FIG. 1 may be implemented in a hardware, a software, or a combination of software and hardware, and they can include one or more signal processing circuits and/or application specific integrated circuits.

The memory 102 may include a high-speed random-access memory and may also include a nonvolatile memory, such as one or more disk storage devices, flash memory devices, or other nonvolatile solid-state storage devices. In some embodiments, the memory 104 may also include a memory which is remote from one or more processers/controllers 150, such as an additional network memory accessed via the RF circuit 120 or via the external interface 122 and communication network (not shown). The communication network may be an Internet, one or more internal networks, a local area network (LAN), a wide area network (WLAN), a storage area network (SAN), etc., or an appropriate combination thereof. The processor/controller 150 may control access to the memory 104 by other components of the medical device 10 other than the peripheral device interface 152.

The peripheral device interface 152 couples input and output peripherals of the medical device 10 to the processor/controller 150 and the memory 104. The one or more processor/controllers 150 run various software programs and/or instruction groups stored in the memory 104 to perform various functions of the medical device 10 and process data.

In some embodiments, the peripheral device interface 152 and the processor/controller 150 may be implemented on a single chip. In some embodiments, they can be implemented on multiple discrete chips.

The RF (radio frequency) circuit 120 receives and transmits electromagnetic waves. The RF circuit 120 converts electrical signals into electromagnetic waves, or converts electromagnetic waves into electrical signals, and communicates with communication networks and other communication devices via electromagnetic waves. The RF circuit 120 may include well-known circuits for performing these functions, and these well-known circuits include but are not limited to the antenna system 156, a RF transceiver, one or more amplifiers, a tuner, one or more oscillators, a digital signal processor, a CODEC chipset, a user identity module (SIM) card, a memory, etc. The RF circuit 120 may communicate with networks and other devices through a wireless communication, which may be the world wide web (WWW), an intranet and/or a wireless network such as a cellular telephone network, a wireless local area network (LAN) and/or a metropolitan area network (MAN). The wireless communication can use any of a variety of communication standards, protocols and technologies, which include but are not limited to the global system for mobile communications (GSM), enhanced data GSM environment (EDGE), wideband code division multiple access (WCDMA), code division multiple access (CDMA), time division multiple access (TDMA), Bluetooth (such as ieee802.15.1), wireless fidelity (WiFi) (e.g.,

IEEE802.11a, IEEE 802.11b, IEEE 802.11g and/or IEEE 802.11n), voice over internet protocol (VoIP), WI-MAX, protocols for e-mail, instant messaging and/or short message service (SMS), or any other suitable communication protocols which include those are not yet developed at the date of submission of this disclosure.

The external interface 122 provides a wired communication interface between the medical device 10, other devices (such as a Dock, central station, monitor, etc.) or users (computers or other communication devices). In some embodiments, it can be a communication interface controlled by a CAN bus protocol, a communication interface controlled by a serial communication protocol (such as RS485, RS232), or a universal serial bus (USB). The external interface 122 is suitable for being directly or indirectly coupled to other devices or users via a network (such as the Internet, LAN, etc.).

The audio circuit 124 and the speaker 154 provide an audio interface between the user and the medical device 10. The audio circuit 124 receives audio data outputted from the peripheral device interface 152 through the output interface, converts the audio data into an electrical signal, and transmits the electrical signal to the speaker 154. The speaker 154 converts the electrical signal into a sound wave that can be perceived by human.

The monitoring circuit 126 may include a fault detection circuit which is operable to instruct a state of one or more processors/controllers 150.

The protection circuit 128 may include a hardware protection device (e.g., fuses, TVS diodes) which is operable to protect the electrical safety of various components in the medical device 10. The processor/controller 150 drives the power device (unshown) of the medical device 10 through the power drive circuit 130, so that the power device can be moved controllably under the drive of the processor/controller 150. In the process of movement, one or more force transmission/conversion devices (such as gears, transmission shafts, screw rods, screw nuts or sliders) drive the controlled object (such as a pump door or a push-pull box) to move. The power device can be an electromagnetic device that realizes the electric energy conversion or transmission according to the law of electromagnetic induction, such as a permanent magnet (PM) motor, a reactive (VR) motor and a hybrid (HB) motor. In some embodiments, the motor is driven by the processor/controller 150 to enable the controlled object of the medical device 10(such as a pump door or a push-pull box) to move, such that a preset movement state of the controlled object can be realized.

In some embodiments, the infusion apparatus is a syringe. The push-pull box is operable to clamp the piston of the syringe. The processor/controller 150 in the medical device 10 sends instructions such as a speed instruction or a movement position instruction, and drives the power device through the power drive circuit 130 to control the injection action of the syringe pump. Specifically, the power device sends a control pulse to rotate the motor through the drive circuit, and the motor drives the screw rod and screw nut through a deceleration mechanism to convert the rotation motion of the motor into a linear motion of the screw nut. The screw nut is connected with the push rod of the matched syringe, and the push rod is connected with the push-pull box. The push-pull box can push the piston of the matched syringe for infusion. By configuring the rotation speed of the motor, the pushing speed of the motor to the matched syringe can be adjusted, such that the given infusion dosage and infusion speed can be adjusted.

In some embodiments, the pressure sensor 136 may respond to a pressure value on the measured object, convert the pressure value into an electrical signal which is detectable and send it to the control platform 102. The pressure sensor 136 may be a resistance strain gauge pressure sensor, a semiconductor strain gauge pressure sensor, a piezoresistive pressure sensor, an inductive pressure sensor, a capacitive pressure sensor, a resonant pressure sensor, an optical fiber pressure sensor, or a capacitive acceleration sensor. In some embodiments, the pressure sensor 136 may be operable to detect an internal pressure of the infusion apparatus or an external pressure of the infusion apparatus. In some embodiments, the pressure sensor 136 can also be operable to detect an in-position state of the measured object (such as the syringe, etc.). In some embodiments, the pressure sensor 136 may detect a blockage in the infusion apparatus or whether the syringe 60 leaks.

In some embodiments, the medical device 10 has a heating device for heating the liquid in the infusion apparatus. At this time, the temperature sensor 138 can be operable to detect the real-time temperature of the liquid. At the same time, the temperature value is converted into an electrical signal which is detectable and then sent to the control platform 102. The control platform 102 can display the real-time temperature through the display system 160 or control an on/off state of the heating device according to the temperature value.

The input/output (I/O) system 108 provides an interface between the input/output peripherals of the medical device 10 and the peripheral device interface 152. The input/output peripherals may be a display system 160, a position sensor 164, a displacement sensor 166, a light component 168, and other input/control devices 162. The I/O system 108 may include a display controller 140, a position sensor controller 144, a proximity sensor controller 146, a light controller 148, and one or more other input controllers 142. One or more controllers in the I/O system 108 receive/transmit electrical signals from/to input/output peripherals. One or more input controllers 142 receive/transmit electrical signals from/to other input/control device 162. The other input/control device 162 may include a physical button (such as, a press button, a rocker button or a touch button, etc.), a slider switch, a joystick, etc. In some embodiments, the other input/control device 162 may include a physical button for emergency stop of infusion.

In some embodiments, the display system 160 may include a display screen that provides an output interface between the medical device 10 and the user. The display screen displays electronic documents on the screen through a specific transmission device and then reflects them to the human eye. The display screen can include a cathode ray pipe display (CRT), a plasma display (PDP) or a liquid crystal display (LCD), etc. In some embodiments, the display system 160 may include a touch screen that provides an input/output interface between the medical device 10 and the user. The touch screen can be an inductive display device that can receive input signals such as contacts, and include a resistance screen, a surface acoustic wave screen, an infrared touch screen, an optical touch screen, a capacitive screen or a nano film. Both of the display screen and touch screen can display the visual output to the user, such as through the output interface in the peripheral device interface 152. Visual output optionally includes graphics, text, chart, video, and combinations thereof. Some or all visual outputs can correspond to user interface objects, which will be described in more detail in this disclosure.

The touch screen also receives an input of user based on touch and/or contact. The touch screen forms a touch sensitive surface that receives the input of user. The touch screen and display controller 140 (together with any associated modules and/or instruction group in the memory 104) detects contact on the touch screen (and any movement or interruption of the touch) and converts the detected contact into an interaction with user interface objects such as one or more software keys displayed on the touch screen. In one exemplary embodiment, the contact point between the touch screen and the user corresponds to one or more fingers of the user. The touch screen can use LCD (liquid crystal display) technology or LPD (light emitting polymer display) technology. However, other display technologies can be used in other embodiments. The touch screen and display controller 140 may use any of a variety of touch sensitive technologies to detect the contact and its movement or interruption, which include but are not limited to capacitance, resistance, infrared and surface acoustic wave technologies, as well as other proximity sensor arrays, or other technologies for determining one or more points of contact with the touch screen.

The position sensor 164 can sense a position of the measured object, convert the position into an electrical signal which is detectable, and send the electrical signal to the control platform 102 through the I/O system 108. The position sensor 164 can be a contact sensor that generates a signal through contact and compression of two objects, such as a travel switch and a two-dimensional matrix position sensor; or can be a proximity sensor that generates a signal when two objects approach in a preset distance, such as an electromagnetic sensor, a photoelectric sensor, a differential transformer sensor, an eddy current sensor, a capacitive sensor, a reed switch sensor, an ultrasonic sensor or a hall sensor. The measured object can include the infusion apparatus, pump door, pump vane, liquid stopping clip and push rod, etc. In some embodiments, a hall position sensor may be operable to detect the position of the pump door. In some embodiments, a photoelectric position sensor may be operable to detect the position of the pump vane. In some embodiments, a photoelectric position sensor can be operable to detect whether the infusion apparatus is arranged at a preset position. In some embodiments, a photoelectric position sensor can be operable to detect the position state of the clamping mechanism for the syringe. In some embodiments, a photoelectric position sensor can be operable to detect the clipping position of the liquid stopping clip on the pipe.

The displacement sensor 166 can respond to a change of position of the measured object relative to a reference position, convert the change of position into an electrical signal which is detectable, and send the electrical signal to the control platform 102 through the I/O system 108. The displacement sensor 106 may be an inductive sensor, a capacitive sensor, an ultrasonic sensor or a hall sensor. In some embodiments, a potentiometer may be operable to monitor the change of position of the pump door. In some embodiments, a potentiometer may be operable to monitor a change of position of a slider of the syringe pump. In some embodiments, a rotary potentiometer may be operable to monitor a change of an outer diameter of an infusion apparatus, such as a syringe.

The light component 168 may include a visual alarm element which indicates that the medical device 10 is in an abnormal state. Light component 168 individually responds to the drive of the processor/controller 150. The light component 168 may also cooperate with the speaker 154 to respond to the drive of the processor/controller 150. For example, the color or brightness of the light changes with the tone and frequency of the alarm sound. The light component 168 may include indicators for a power supply, a CPU and other components or an alarm indicator which indicates the infusion failure state. The light component 168 may also include a visual lighting element for facilitating the observation of the structure or component state of the medical device 10 when the ambient light is poor.

The medical device 10 also includes a power supply system 106 for supplying power to various components. The power system 106 may include a power management system, one or more power sources (e.g., batteries or alternating current (AC)), a charging system, a power failure detection circuit, a power converter or inverter, a power state indicator (e.g., light emitting diodes (LEDs)), or any other component associated with power generation, management and distribution.

In some embodiments, the software components include an operation system 170, a communication module (or instruction group) 172, a touch module (or instruction group) 174, a tactile feedback module (or instruction group) 176, a movement module (or instruction group) 178, a position module (or instruction group) 180, a graphics module (or instruction group) 182, a text input module (or instruction group) 190, a device/overall internal state (or instruction group) 192, and one or more applications (instruction group) 194.

Operation system 170 (e.g., embedded operation systems, such as Darwin, RTXC, LINUX, UNIX, OS, WINDOWS, etc.) includes various software components and/or drivers for controlling and managing conventional system tasks (e.g., memory management, storage device control, power management, etc.) and facilitating communication between various software and hardware components.

The communication module 172 facilitates to communicate with other devices via one or more external interfaces 122, and it also includes various software components which are operable to process data received by the RF (radio frequency) circuit 120 and/or the external interface 122.

In some embodiments, the touch module 174 may selectively detect contact with the display system 160 or other touch sensitive devices (e.g., touch buttons, touch pads). For example, the touch module 174 detects contact with the display system 160 together with the display controller 140. The touch module 174 includes various software components which are operable to perform various operations associated with contact detection of the display system 160 (which can be implemented by a finger or a stylus, etc.), such as operations of determining whether the contact occurs (e.g., detecting a press time of a finger), determining a strength of the contact (e.g., a force or pressure of the contact), determining whether the contact moves (e.g., detecting one or more finger dragging events), tracking the movement on the display, and determining whether the contact stops (e.g., detecting a lift time of the finger or a disconnection of the contact). Wherein the operation of determining a movement of a contact point may include determining a rate (amplitude), a speed (amplitude and direction), and/or an acceleration (including amplitude and/or direction) of the contact point. These operations can be applied to single-point contact or multi-point simultaneous contact. In some embodiments, the touch module 174 detects contact with other touch devices in conjunction with the display controller 140.

The touch module 174 may be operable to detect gesture inputted by the user. Different gestures of the user on the touch sensitive device have different contact modes (for example, a detected contact position, contact time or contact intensity, or combinations of one or more thereof). For example, detecting gesture of tapping by a single finger includes detecting a finger press event, and then detecting a finger lift event at the same or similar position as the finger press event. For example, detecting gesture of swiping by a finger includes detecting a finger press event, and then monitoring one or more finger dragging events, and finally detecting a finger lift event. Similarly, the gestures of the touch pen, such as tapping, swiping, dragging and so on, are optionally detected by detecting a designated contact pattern of the touch pen.

The tactile feedback module 176 includes various software components which are operable to generate instructions for generating a tactile output at one or more positions of the medical device 10 by using one or more tactile output generators (not shown), in response to the interaction of the user with the medical device 10. For example, after detecting the contact on the surface of the touch device, the color of the pattern or text of the touch device changes, or a sound or vibration generates.

The position module 180 includes software components which are operable to perform various operations which are related to the detection of the device position and the detection of the change of the device position.

The graphics module 182 includes various known software components which are operable to render or display graphics on the display screen of the display system 160 or other external devices, and includes components which are operable to change the visual impact (e.g., brightness, transparency, saturation, contrast or other visual attributes) of the displayed graphics. In the embodiments herein, the term "graphics" includes any object that can be displayed to the user, which includes but is not limited to text, web page, icon (e.g., user interface objects of soft keys), digital image, video, animation, and the likes. In some embodiments, the graphics module 182 stores data which represents the graphics to be used. Each graphics can be assigned a corresponding code. The graphics module 182 receives one or more codes for the graphics which is designated to be displayed from an application or the like, and also receives coordinate data together with other graphics attribute data if necessary, and then generates screen image data for outputting to the display controller 140.

The text input module 190 provides various software components which are operable to input text into one or more applications. Specifically, it can be used to input various infusion parameters, which include a medicine name, infusion speed or alarm threshold.

In some embodiments, the memory 104 stores the device/overall internal state 192. The device/overall internal state 192 includes one or more of the followings: an active application state that indicates which applications (if any) are currently active; a display state that indicates what applications, views, or other information occupy various areas of the display system 160; a sensor state that includes information obtained from each sensor of the device and other input controllers 142; and position and/or orientation information about a position and/or posture of the device.

In some embodiments, the memory 104 (FIG.1) stores at least one application 194, which may include an infusion mode setting 194-1, a blocking pressure level setting 194-2, a medicine setting 194-4, a volume setting 194-5, a brightness setting 194-6, an online setting 195-7, a Dock setting 195-8, or a temperature setting 195-9. The infusion mode setting 194-1 can include a combination of preset infusion parameters for satisfying requirements of different use scenarios. The blocking pressure level setting 194-2 can include an interface for the user to input different blocking pressure levels. By inputting different blocking pressures, the blocking alarm threshold of the medical device 10 can be adjusted for satisfying requirements of different use scenarios. The medicine setting 194-4 can include an interface for the user to input different medicine names, medicine abbreviations and/or medicine colors. The medicine parameters which are set before the infusion by inputting the corresponding medicine names/abbreviations/colors, can facilitate the automatic confirmation inside the medical device 10 or the verification of medical staff during infusion. The volume setting 194-5 allows the user to adjust the volume of alarm and/or other audio output according to their requirements. The brightness setting 194-6 allows the user to adjust the brightness of the screen, alarm indicator, lighting lamp and so on, according to their requirements. The online setting 195-7 provides an input interface for the user to control the medical device 10 to work online with other devices or not and to control an online working mode according to their requirements. The Dock setting 195-8 provides a setting interface for the user to adjust the working parameters of the Dock connected to the medical device 10 according to their requirements. The temperature setting 195-9 provides a setting interface for the user to heat the liquid in the infusion apparatus.

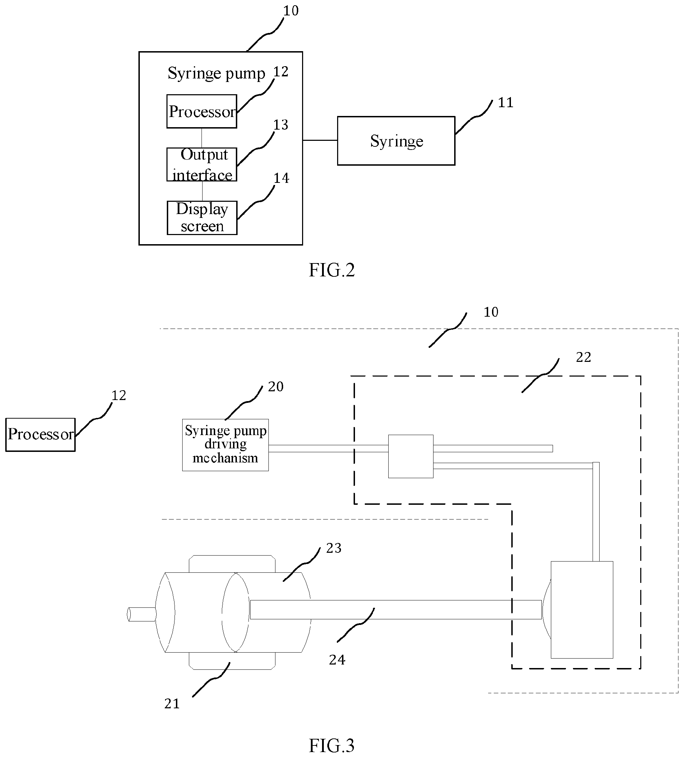

Wherein, the medical device 10 provided above can be a syringe pump. The syringe pump which is provided by the embodiment of this disclosure can be applied to this disclosure environment shown in FIG. 2. The syringe pump 10 is clamped and connected with the syringe 11, and the syringe pump 10 can push the piston in the syringe 11 to move relative to each other. The syringe pump 10 includes a processor 12. Alternatively, the processor 12 can be a CPU (central processing unit), IPU (intelligence processing unit), etc. The syringe pump 10 also includes an output interface 13 and a display screen 14, and the processor 12 is connected with the display screen 14 through the output interface 13. The displacement of the piston can be acquired according to the relative movement between the piston and the body of the syringe. Then the medicine amount information can be determined according to the displacement of the piston and the syringe model information which is inputted by the user, through the processor 12.

The display screen 14 provides an output interface between the syringe pump 10 and the user, and displays the medicine amount information on the screen through a specific transmission device and then reflects it to the human eye from the display screen. The display screen can include a cathode ray pipe display (CRT), a plasma display (PDP) or a liquid crystal display (LCD), etc. Visual output optionally includes graphics, text, chart, video, and combinations thereof. Some or all visual outputs can correspond to user interface objects, which will be described in more detail in this disclosure.

In some embodiments, as shown in FIG.3, a structural block diagram of a syringe pump 10 is provided. The syringe pump 10 includes a syringe pump drive mechanism 20, a syringe clamping mechanism 21, a syringe propulsion mechanism 22, a processor 12, an output interface and a display screen.

The syringe clamping mechanism 21 is operable to clamp a body 23 of a syringe, the syringe pump drive mechanism 20 is operable to drive the syringe propulsion mechanism 22 to push a piston 24 of the syringe for enabling a relative movement between the body 23 and the piston 24 of the syringe. The processor 12 is operable to connect with the display screen through the output interface and is operable to display medicine amount information on the display screen after the syringe is clamped by the syringe clamping mechanism 21 and syringe model information is inputted by a user. The medicine amount information changes along with operation of the syringe pump drive mechanism 20.

The syringe model information can be syringe brand information, or can be both of syringe brand information and syringe model information. For example, if the syringe brand includes only one model, the size information of the syringe can also be acquired through the syringe brand information. If the syringe brand includes a plurality of models, the size information of the syringe may be determined by identifying both of the syringe brand information and syringe model information (such as 50ml).

As shown in FIG. 3, the syringe propulsion mechanism 22 can push the piston 24 to move relative to the body 23 of the syringe. The processor 12 calculates the displacement of the piston 24 according to the relative movement, and then determines the medicine amount information according to the syringe model information which is inputted by the user and the displacement of the piston 24. The processor 12 displays the medicine amount information on the display screen through the output interface.

The value of the medicine amount information gradually decreases with the operation of the syringe pump drive mechanism 20.

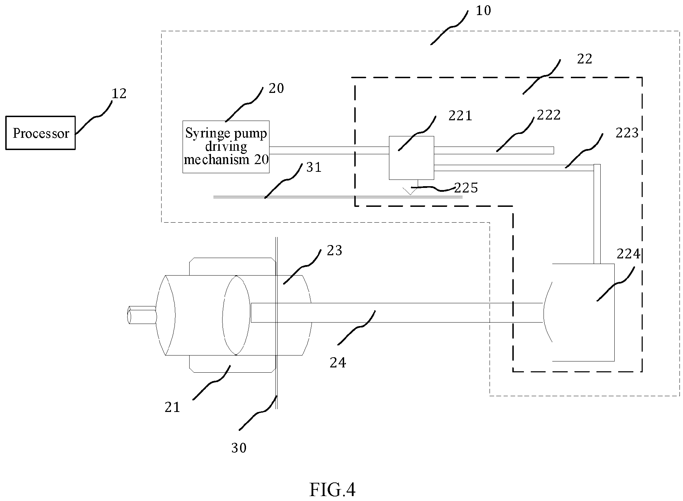

In some embodiments, as shown in FIG. 4, a structural block diagram of a syringe pump 10 is provided, which includes a first sensor 30 and a second sensor 31 in addition to the components shown in FIG. 3.

The first sensor 30 is associated with the syringe clamping mechanism 21, and the processor 12 is operable to determine a radial length of the syringe according to a feedback signal of the first sensor 30. The second sensor 31 is associated with the syringe propulsion mechanism 22, and the processor 12 is operable to determine a displacement of the piston 24 relative to the body 23 of the syringe according to a feedback signal of the second sensor 31. The processor 12 is also operable to determine the medicine amount information according to the radial length of the syringe and the displacement.

In some embodiments, the first sensor 30 includes a rotary potentiometer, a Hall sensor, or a magnetoresistive sensor.

In some embodiments, the second sensor 31 includes a slide potentiometer, a film potentiometer, a grating ruler or a capacitive grating ruler.

Wherein, the association between the first sensor 30 and the syringe clamping mechanism 21 indicates that the feedback signals of the first sensor 30 change accordingly with the change of an opening and closing state of the syringe clamping mechanism 21. It can be understood that the feedback signals of the first sensor 30 characterize the opening and closing size of the syringe clamping mechanism 21. When the syringe clamping mechanism 21 clamps the syringe, the opening and closing size is equivalent to the radial length of the syringe. The association between the second sensor 31 and the syringe propulsion mechanism 22 indicates that the feedback signals of the second sensor 31 change accordingly with the movement of the syringe propulsion mechanism 22. It can be understood that, the feedback signals of the second sensor 31 characterize the displacement distance of the syringe propulsion mechanism 22. When the syringe propulsion mechanism 22 pushes the piston 24 of the syringe to move relative to the body 23 of the syringe, the displacement distance of the syringe propulsion mechanism 22 is equivalent to the displacement of the piston 24 relative to the body 23 of the syringe. In the embodiment of this disclosure, the syringe propulsion mechanism 22 includes a slider 221, a pump body screw 222, a push rod 223, a push-pull box 224 and a plunger 225. Wherein the slider 221 is connected with the plunger 225. The slider 221 and the piston 24 move in the same direction with an equivalent distance. The movement with an equivalent distance means that the displacement of the slider 221 is equivalent to the displacement of the piston 24. The plunger 225, which is connected with the slider 221, slides on a surface of the second sensor 31. When the syringe is installed, the displacement of the slider 221 is equivalent to the displacement of the piston 24, and meanwhile the displacement of the slider 221 is also equivalent to the displacement of the plunger 225 on the second sensor 31. Since the displacement of the plunger 225 on the second sensor 31 is linearly related to its output voltage, the displacement of the piston 24 can be directly acquired by using the output of the second sensor 31. The length of the body of the syringe can be determined according to the syringe model information. Then, the processor 12 determines the length of the remaining medicine amount, according to the displacement of the piston 24 and the length of the body of the syringe, and then acquires the medicine amount information according to the length of the remaining medicine amount and the radial length of the syringe. Among them, the medicine amount information is the residual capacity of the medicine.

In this embodiment, the radial length of the syringe can be measured in real time by arranging the first sensor 30, and then the real-time measured radial length can be used for subsequent calculations. In this way, the medicine information can be acquired more accurately. In addition, through the arrangement of the first sensor 30, when the user sets the infusion parameters, the processor can directly obtain the radial length of the syringe through the first sensor 30 and instruct the user to select on the display screen for facilitating the operations of the user.

In some embodiments, the syringe pump also includes a memory and a second sensor.

The memory is operable to store a preset radial length of the syringe corresponding to the syringe model. The second sensor is associated with the syringe propulsion mechanism, and the processor is operable to determine the displacement of the piston relative to the body of the syringe according to a feedback signal of the second sensor. The processor is operable to retrieve the radial length of the syringe stored in the memory and determine the medicine amount information according to the radial length of the syringe and the displacement.

The radial length of the syringe which is corresponding to the syringe model information is preset in the memory, so there is no need to set the first sensor as in the embodiment shown in FIG. 4.

The method for acquiring the displacement of the piston by using the second sensor is consistent with that described in the embodiment shown in FIG. 4, which will not be repeated here.

After the processor determines the length of the body of the syringe according to the syringe model information, the processor 12 determines the length of the remaining medicine amount, according to the displacement of the piston 24 and the length of the body of the syringe, and then acquires the medicine amount information according to the length of the remaining medicine amount and the radial length of the syringe which is stored in the memory and retrieved out by the processor.

In some embodiments, in order to determine the displacement of the piston relative to the body of the syringe according to the feedback signal of the second sensor, the processor is specifically operable to determine a start position of the piston and a current position of the piston according to the feedback signal of the second sensor, and determine the displacement of the piston relative to the body of the syringe according to the start position of the piston and the current position of the piston.

As shown in FIG. 4, the length of the body 23 of the syringe (recorded as Li) is acquired according to the syringe model information. Then, after installing the syringe filled with a liquid medicine into the syringe pump, the push-pull box 224 of the syringe is pushed to a position that just contacts a hand-pressing position of the syringe. Wherein the hand-pressing position of the syringe is the position at the handle). Then position of the push-pull box 224 at this time is recorded as the start position of the piston 24 through the second sensor 31. Then, the piston 24 is pushed to inject the liquid medicine into the patient (in this process, the push-pull box 224 always just contacts the hand-pressing position of the syringe), the current position of the piston 24 is recorded through the second sensor 31 in real time. The displacement of the piston 24 relative to the body 23 of the syringe, which is recorded as $L_2$, is obtained by subtracting the current position from the start position. Then, the length of the remaining medicine amount L is equivalent to $L_1$-$L_2$. Then the medicine amount information is acquired according to the radial length of the syringe and the remaining medicine amount L.

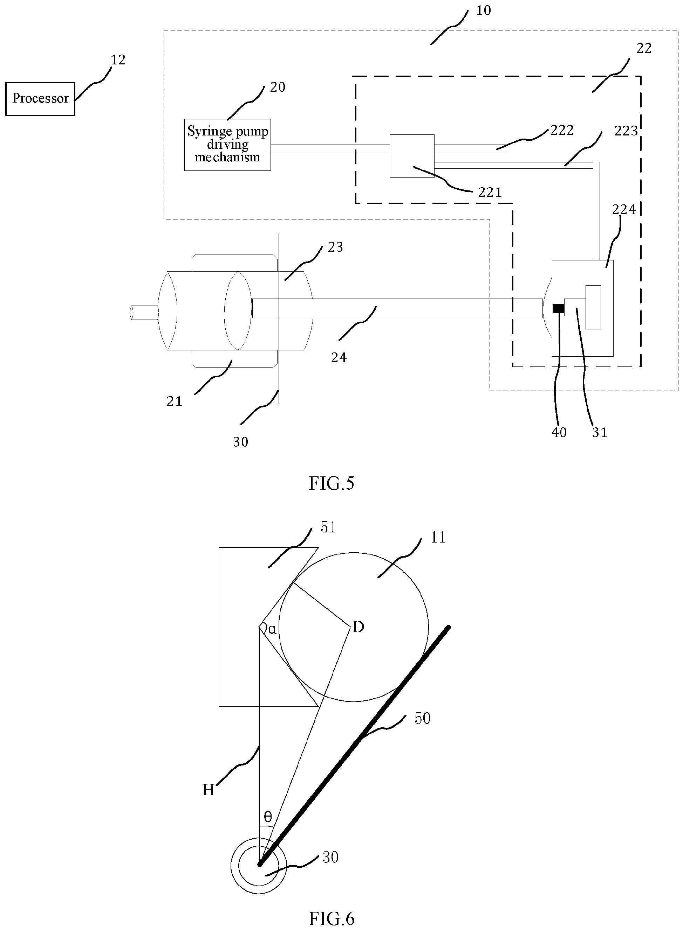

In some embodiments, as shown in FIG. 5, a structural block diagram of a syringe pump 10 is provided, which includes a first sensor 30 and a second sensor 31 in addition to the components shown in FIG. 3.

The first sensor 30 is associated with the syringe clamping mechanism 21, that is, a feedback signal of the first sensor 30 is associated with an opening and closing degree of the syringe clamping mechanism 21. The processor 12 is operable to determine a radial length of the syringe according to the feedback signal of the first sensor 30. The second sensor 31 is associated with the syringe propulsion mechanism 22, that is, a feedback signal of the second sensor 31 is associated with an activation of a travel distance of the syringe propulsion mechanism 22.

In some embodiments, the first sensor includes a rotary potentiometer, a Hall sensor, or a magnetoresistive sensor.

In some embodiments, the second sensor 31 includes an optocoupler, a travel switch, a contact switch or a Hall sensor.

The processor 12 is operable to determine the start position of the piston 24 according to the feedback signal of the second sensor 31, determine the displacement of the piston 24 relative to the body 23 of the syringe according to the start position of the piston 24 and the number of revolutions of the motor.

It should be noted that this embodiment can be applied to the non-clutch type syringe pump with a screw rod and screw nut.

As shown in FIG. 5, a detection bump for a hand-pressing position of the syringe, is added in the middle direction of a push handle of the push-pull box 224, and a second sensor 31 is arranged behind the bump.

Among them, the processor acquires the length of the body 23 of the syringe (recorded as $L_1$) according to the syringe model information. Then the syringe filled with a liquid medicine is installed on the syringe pump 10. The controller drives the motor to just move to a position where the detection bump for a hand-pressing position of the syringe rightly triggers the second sensor 31, and then the start position of the piston is recorded at this time. After that, the piston 24 is pushed to inject the liquid medicine into the patient, and the number of revolutions of the drive shaft of the motor at this time is recorded as R. According to the number of revolutions of the drive shaft of the motor, the current position of the

$$\Delta L = \frac{R}{N} \times S$$

piston can be calculated as , $0 \le \Delta L \le L_T$. Wherein, N refers to a gear reduction ratio between the motor and the pump body screw 222, S refers to a lead of the pump body screw 222, and $L_T$ refers to a total length of the screw. The displacement of the piston 24 relative to the body 23 of the syringe, which is recorded as $L_2$, is obtained by subtracting the current position from the start position. Then, the length of the remaining medicine amount L is equivalent to $L_1$-$L_2$. Then the medicine amount information is acquired according to the radial length of the syringe and the length of the remaining medicine amount L.

The second sensor 31 can sense a position of the measured object, convert the position into an electrical signal which is detectable, and send the electrical signal to the processor 12 through the I/O system. The second sensor 31 can be a contact sensor that generates a signal through contact and compression of two objects, such as a travel switch and a two-dimensional matrix position sensor; or can be a proximity sensor that generates a signal when two objects approach in a preset distance, such as an electromagnetic sensor, a photoelectric sensor, a differential transformer sensor, an eddy current sensor, a capacitive sensor, a reed switch sensor, an ultrasonic sensor or a Hall sensor. In the embodiments of the present disclosure, the measured object can include a blocking vane or a detection bump for a hand-pressing position of the syringe.

The above scheme describes in detail how to determine the displacement of the piston 24 relative to the body 23 of the syringe 11. How to determine the radial length of the syringe 11 will be described in detail below. In some embodiments, as shown in FIG. 6, the radial length of the syringe 11 is calculated by using the first sensor 30. The clamping arm 50 of the syringe 11 drives the first sensor 30 to rotate. The outputted voltage signal of the first sensor 30 is linearly related with the rotation angle. Therefore, the rotation angle θ of the clamping arm 50 of the syringe 11 can be obtained according to the outputted signal of the first sensor 30. The syringe 11 is installed on the syringe pump, and is limited by the structural fastener 51 on the body of the syringe pump. The vertical distance H from the center of the syringe 11 to the first sensor 30 is a fixed value, and an opening angle of the structural fastener 51 on the body of the syringe pump is a fixed value α. Then, according to the geometric relationship, the radial length D of the syringe 11 is calculated as:

$$D = 2 \times H \times \tan\theta \times \sin\frac{\alpha}{2} - 2 \times d \; .$$

Wherein, D refers to a radial length of the syringe 11, H refers to a vertical distance from a center of the syringe 11 to the first sensor 30, $\theta$ refers to a rotation angle of the clamping arm 50 of the syringe 11, $\alpha$ refers to an opening angle of the structural fastener 51 on the body of the syringe pump, and d refers to a wall thickness of the syringe 11 which is installed the syringe pump.

According to the scheme in the above embodiment, the radial length D and the length of the remaining medicine amount L of the syringe 11 can be obtained, and the remaining medicine amount (i.e., the medicine amount information) can be obtained according to the following formula:

$$V = \pi \times \left(\frac{D}{2}\right)^2 \times L \; ;$$

wherein V refers to the medicine amount information, D refers to the radial length of the syringe 11, L refers to the length of the remaining medicine amount.

In some embodiment, the syringe pump also includes a memory which is operable to store an output function of the first sensor, and the output function characterizes a functional relationship between the feedback signal of the first sensor and the radial length of the syringe. The processor is operable to determine the medicine amount information according to the output function, the radial length of the syringe and the displacement.

Wherein, there may be errors in the detection of the radial length of the syringe. The errors are mainly composed of a mechanical dimensional error of the clamping arm of the syringe, a linear error of the second sensor (for example, the linear error of the rotary potentiometer). The syringe pump can eliminate these errors by detecting and calibrating the radial length of the syringe before leaving the factory. Specifically, two syringe-like standard parts with a fixed length shall be used for calibration. That is, two standard parts are installed respectively, and then the syringe pump shall record the corresponding detection values of the two standard parts. The detection values are compared with two parameters of $D_a$ and $D_b$ for correcting the detection function for the syringe diameter built-in the syringe pump (i.e., the output function of the first sensor). Then, according to the output function of the first sensor, the radial length of the syringe and the displacement, the medicine amount information can be determined more accurately.

In some embodiment, the syringe pump also includes a memory for storing an output function of the second sensor, and the output function characterizes a functional relationship between the feedback signal of the second sensor and the displacement of the piston of the syringe. The processor is operable to determine the medicine amount information according to the output function, the radial length of the syringe and the displacement.

In the embodiment of this disclosure, there may be errors in the detection of the pull-out length of the syringe. The errors are mainly composed of a lead error of the screw rod of the pump body, an error in a gear meshing, a linear error of the second sensor (for example, a linear error of the slide potentiometer), and the rotation error of the motor. The syringe pump can eliminate these errors by detecting and calibrating the pull-out length of the syringe before leaving the factory. Specifically, two syringe-like standard parts with a fixed length ($L_a$, $L_b$) shall be installed on the syringe pump respectively, and then the push-pull box shall be pushed to just contact a hand-pressing position of the standard part. The syringe pump shall record the corresponding detection values of the two standard parts for correcting the detection function for the pull-out length of the syringe built-in the syringe pump (i.e., the second sensor, the radial length of the syringe and the displacement, the medicine amount information can be determined more accurately.

In some embodiments, the medicine amount information in the syringe pump is determined according to the output function of the first sensor, the output function of the second sensor, the radial length of the syringe and the displacement.

Wherein, the processor calibrates the radial length of the syringe according to the output function of the first sensor to obtain the calibrated radial length of the syringe, calibrates the displacement of the piston according to the output function of the second sensor to obtain the calibrated displacement of the piston, and then determine the medicine amount information according to the calibrated radial length of the syringe and the calibrated displacement of the piston, more accurately.

In some embodiments, the syringe pump includes a syringe pump drive mechanism, a syringe clamping mechanism, a syringe propulsion mechanism, a processor, an output interface and a display screen. The syringe clamping mechanism is operable to clamp a body of a syringe. The syringe pump drive mechanism is operable to drive the syringe propulsion mechanism to push a piston of the syringe for enabling a relative movement between the body of the syringe and the piston. The processor is operable to connect with the display screen through the output interface and is operable to display injection time information on the display screen, after the syringe is clamped by the syringe clamping mechanism and syringe model information and a flow rate are inputted by a user. Wein the injection time information changes along with operation of the syringe pump drive mechanism.

The injection time information is the remaining infusion time (that is, the remaining time), and the injection time gradually decreases with the operation of the syringe pump drive mechanism 20.

In the embodiment of this disclosure, after determining the medicine amount information, the remaining injection time T (i.e., the injection time information X) can be calculated, in combination with the flow rate inputted by the user:

$$T = \frac{V}{S};$$

wherein the medicine amount information is a kind of volume information, so is expressed as V, and S refers to the inputted flow rate.

Among them, the related description of the medicine amount information is consistent with that in the above embodiments, and will not be repeated here.

In some embodiments, the syringe pump also includes a first sensor and a second sensor. The first sensor is associated with the syringe clamping mechanism, and the processor is operable to determine the radial length of the syringe according to the feedback signal of the first sensor. The second sensor is associated with the syringe propulsion mechanism, and the processor is operable to determine the displacement of the piston relative to the body of the syringe according to the feedback signal of the second sensor. The processor is also operable to determine the injection time information according to the radial length, displacement and flow rate of the syringe.

In some embodiments, the first sensor includes a rotary potentiometer, a Hall sensor, or a magnetoresistive sensor. The second sensor includes a slide potentiometer, a film potentiometer, a grating ruler or a capacitive grating ruler.

The related descriptions of the displacement of the piston relative to the body of the syringe, the radial length of the syringe and the injection time information are consistent with those described in the above embodiments, and will not be repeated here.

In some embodiments, the syringe pump also includes a memory for storing the output function of the first sensor, and the output function characterizes the functional relationship between the feedback signal of the first sensor and the radial length of the syringe. The processor is operable to determine the injection time information according to the output function, the radial length, displacement and flow rate of the syringe.

The related descriptions of the output function, the radial length of the syringe, the displacement, the flow rate and injection time information are consistent with those described in the above embodiments, and will not be repeated here.

In some embodiments, the syringe pump also includes a memory and a second sensor. The memory is operable to store a preset radial length of the syringe corresponding to the syringe model. The second sensor is associated with the syringe propulsion mechanism, and the processor is operable to determine the displacement of the piston relative to the body of the syringe according to the feedback signal of the second sensor. The processor is operable to retrieve the radial length of the syringe stored in the memory and determine the injection time information according to the radial length of the syringe, the displacement and the flow rate.

Among them, the related descriptions of the radial length, displacement, flow rate and injection time information of the syringe are consistent with those described in the above embodiments, and will not be repeated here.

In some embodiments, the processor is operable to determine the displacement of the piston relative to the body of the syringe according to the feedback signal of the second sensor, which specifically includes follows. The processor is operable to determine a start position of the piston and a current position of the piston according to the feedback signal of the second sensor, and to determine a displacement of the piston relative to the body of the syringe according to the start position and the current position of the piston.

The related descriptions of the start position of the piston, the current position of the piston and injection time information are consistent with those described in the above embodiments, and will not be repeated here.

In some embodiments, the processor is operable to determine the displacement of the piston relative to the body of the syringe according to the feedback signal of the second sensor, which specifically includes follows. The processor is operable to determine a start position of the piston according to the feedback signal of the second sensor, and to determine a displacement of the piston relative to the body of the syringe according to the start position and a number of revolutions of the motor.

Among them, the related descriptions of the start position of the piston, the number of revolutions of the motor and the injection time information are consistent with those described in the above embodiments, and will not be repeated here.

In some embodiments, the syringe pump also includes a memory for storing an output function of the second sensor, and the output function characterizes the functional relationship between the feedback signal of the second sensor and the displacement of the piston of the syringe. The processor is operable to determine the injection time information of the syringe pump according to the output function, the radial length of the syringe and the displacement.

The related descriptions of the output function, the radial length of the syringe, the displacement, the flow rate and injection time information are consistent with those described in the above embodiments, and will not be repeated here.

In some embodiments, the syringe pump also includes a memory for storing an output function of the first sensor, and the output function characterizes the functional relationship between the feedback signal of the first sensor and the radial length of the syringe. The processor is operable to determine the injection time information according to the output function, the radial length of the syringe, the displacement and flow rate.

Wherein the processor determines the medicine amount information according to the output function, the radial length of the syringe and the displacement, and then determines the injection time information according to the medicine amount information and the flow rate.

Among them, the related descriptions of the medicine amount information is consistent with that in the above embodiments, and will not be repeated here.

In some embodiments, the syringe pump also includes a memory for storing an output function of the second sensor, and the output function characterizes the functional relationship between the feedback signal of the second sensor and the displacement of the piston of the syringe. The processor is operable to determine the injection time information of the syringe pump according to the output function, the radial length of the syringe and the displacement.

Wherein the processor determines the medicine amount information according to the output function, the radial length of the syringe and the displacement, and then determines the injection time information according to the medicine amount information and the flow rate.

In some embodiments, the processor determines the injection time information according to the output function of the first sensor, the output function of the second sensor, the radial length of the syringe, the displacement and the flow rate.

Wherein the processor determines the medicine amount information according to the output function, the radial length of the syringe and the displacement, and then determines the injection time information according to the medicine amount information and the flow rate.

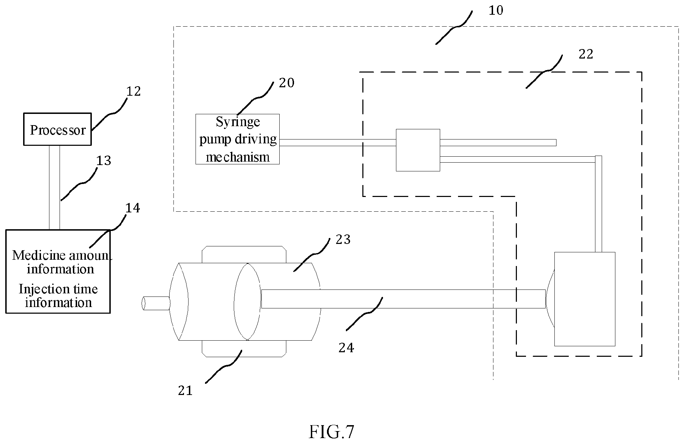

In some embodiments, as shown in FIG.7, a structural block diagram of a syringe pump 10 is provided, which includes a syringe pump drive mechanism 20, a syringe clamping mechanism 21, a syringe propulsion mechanism 22, a processor 12, an output interface 13 and a display screen 14.

The syringe clamping mechanism 21 is operable to clamp a body of a syringe, the syringe pump drive mechanism 20 is operable to drive the syringe propulsion mechanism 22 to push a piston 24 of the syringe for enabling a relative movement between the body 23 of the syringe and the piston 24 of the syringe. The processor 12 is operable to connect with the display screen 14 through the output interface 13 and is operable to display medicine amount information and/or injection time information on the display screen 14 after the syringe is clamped by the syringe clamping mechanism 21 and syringe model information, as well as a flow rate, are inputted by a user. Wherein the medicine amount information and/or the injection time information changes along with operation of the syringe pump drive mechanism 20.

Wherein the medicine amount information or the injection time information is displayed in a first display mode. A ratio between the medicine amount information and a total infusion amount, or a contrast between the injection time information and a total infusion time, is displayed in a second display mode. Wherein the first display mode or the second display mode includes text, number or graphics or combination thereof, and the first display mode is different from the second display mode.

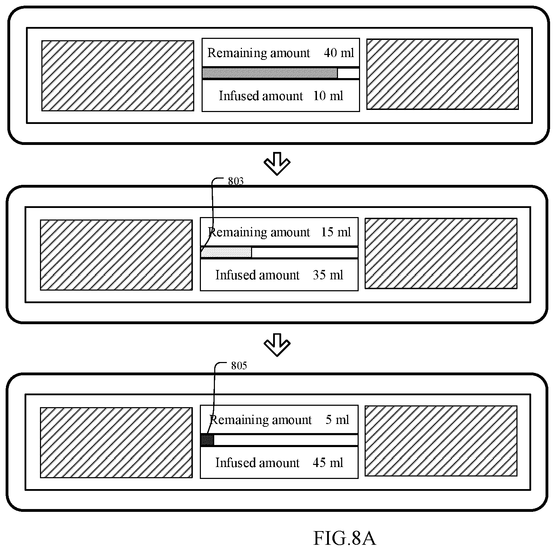

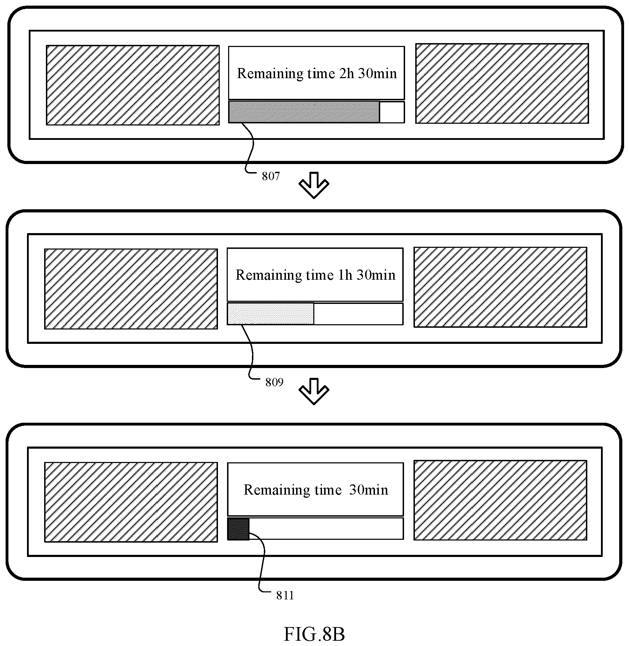

That, the first display mode is different from the second display mode, includes the first display mode is different from the second display mode in types or colors. When the graphics are progress bars for indication, the specific value of the medicine amount information (the residual amount) is displayed on the display interface of the syringe pump. In addition, the progress bar for indication of the medicine amount information is also displayed on the same interface. When the medicine amount information or the injection time information is greater than a first preset threshold, the color of the progress bar for indication is a first preset color. When the medicine amount information or the injection time information is less than the first preset threshold but greater than a second preset threshold, the color of the progress bar for indication is a second preset color. When the medicine amount information or the injection time information is less than the second preset threshold, the color of the progress bar for indication is a third preset color. The first preset color, the second preset color and the third preset color can be different in hue, brightness or saturation. For example, as shown in FIG. 8A, when the medicine amount information (40ml) is greater than the first preset threshold (30ml), the color of the progress bar for indication is the first preset color (e.g., green 801). When the medicine amount information (15ml) is less than the first preset threshold (30ml) but greater than the second preset threshold (10ml), the color of the progress bar for indication is the second preset color (e.g., yellow 803). When the medicine amount information (5ml) is less than the second preset threshold (10ml), the color of the progress bar for indication is the third preset color (e.g., red 805). Of course, the processor can adjust the color or size of the progress bar from other dimensions of the medicine amount information (such as the proportion of the remaining amount to the total infusion amount). As shown in FIG. 8B, when the injection time information (2 hours and 30mins) is greater than the first preset threshold (2 hours), the color of the progress bar for indication is the first preset color (e.g., green 807). When the injection time information (1 hour and 30mins) is less than the first preset threshold (2 hours) but greater than the second preset threshold (1 hour), the color of the progress bar for indication is the second preset color (e.g., yellow 809). When the injection time information (30 mins) is less than the second preset threshold (1 hour), the color of the progress bar for indication is the third preset color (e.g., red 811). Of course, the processor can adjust the color or size of the progress bar from other dimensions of the injection time information (such as the proportion of the remaining time to the total infusion time).

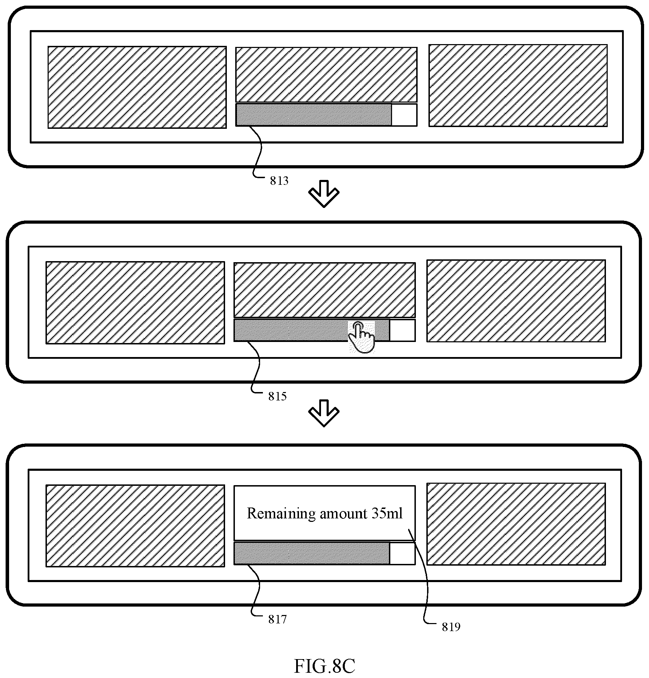

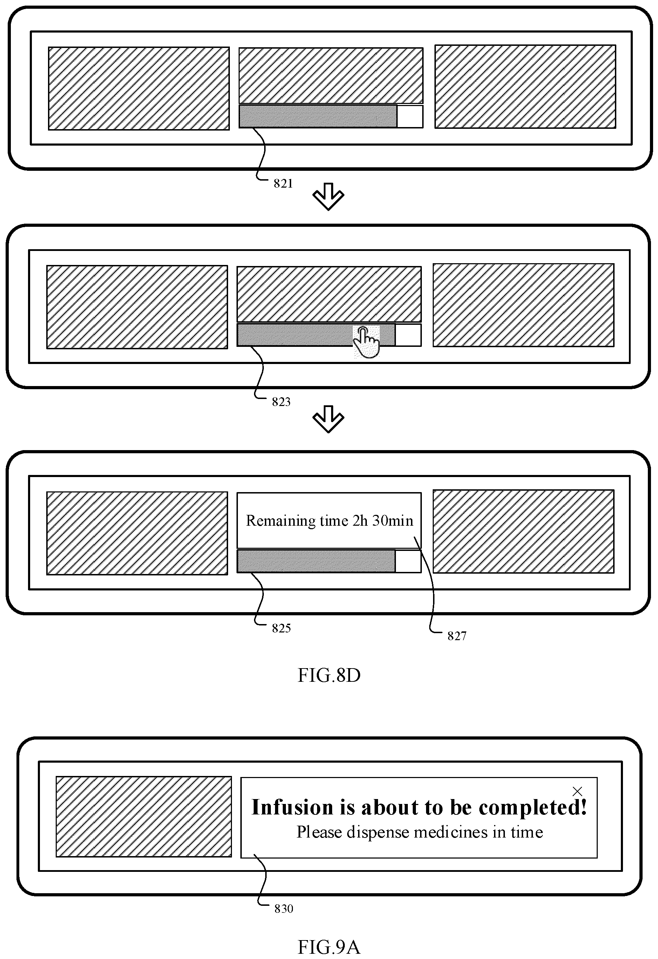

In some embodiments, the processor can also only display a display mode including the medicine amount information or the injection time information in the display interface. As shown in FIGS. 8C and 8D, the processor can only display the progress bar for indication of the remaining amount or the progress bar for indication of the remaining time in the display interface. In some embodiments, the processor can also recognize the gesture input or contact of the user at the position of the progress bar for indication in the display interface. At this time, the processor can also display the remaining amount or remaining time in another display mode on the display interface in detail (819 or 827). Of course, in some embodiments, the processor can also recognize the gesture input or contact of the user at the position of the progress bar for indication in the display interface. At this time, the processor can also switch to another display interface, which only displays the remaining amount or the remaining time in detail in another display mode.

In some embodiments, the processor sends a reminder signal when the medicine amount information and/or the injection time information satisfies the preset conditions.

Among them, when the medicine amount information and/or the injection time information is less than the third preset threshold (for example, the third preset threshold is 3ml), the processor will send a reminder signal. Specifically, the processor triggers the display screen, speaker, alarm light, etc. to send out a reminder signal to remind that the medicine amount is insufficient and needs to be changed. For example, as shown in FIG. 9A, the information that "Infusion is about to be completed! Please dispense medicines in time" is displayed on the display screen, in which the display position of the reminder information can be displayed in a prominent display color, such as red. In some embodiments, the user can close the display frame of the reminder information by touching or contacting other controls on the display interface, so as not to block other important infusion information of the syringe pump.

Among them, the preset conditions are set according to the time for the medical staff to prepare a dosage of medicines. When the medicine amount information and/or the injection time information satisfies the preset conditions, there are still some remaining amount of medicine to infuse the patient. During this period of infusing patients with the remaining amount of medicine, the medical staff can complete the preparation for the dosage of medicines.

Among them, the alarm lamp can respond to the drive of the processor alone, or can also cooperate with the speaker to respond to the drive of the processor. For example, the color or brightness of the light changes with the tone and frequency of the alarm sound.

In some embodiments, the processor determines an infusion end time according to the injection time information and the current time. When a difference between the infusion end time and a preset time for work shift is less than the third preset threshold, the processor will send a reminder signal before the preset time for work shift.

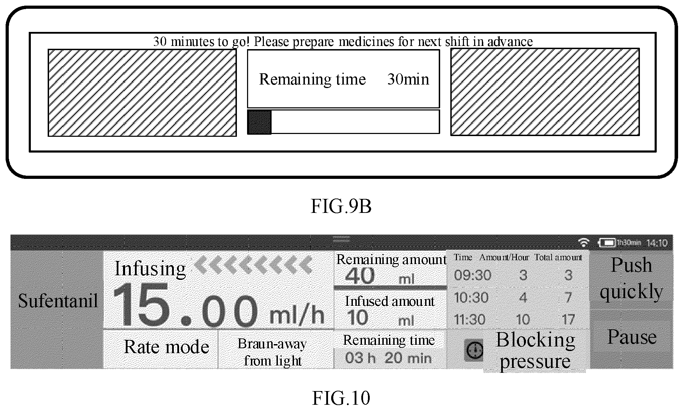

Among them, the processor determines the infusion end time according to the injection time information and the current time. By comparing a difference between the infusion end time and the preset time for work shift with the third preset threshold, the processor determines whether the infusion end time is close to the preset time for work shift. When the infusion end time is close to the preset time for work shift, the processor will send a reminder signal to remind the preparation of medicine before the work shift, as shown in FIG.9B.

In some embodiments, the display interface of the syringe pump can be shown in FIG. 10, in which the display interface displays the injection time information (remaining time) and the medicine amount information (remaining amount), and also displays, in the same interface, the important parameters in the injection process of the syringe pump, such as name of the medicine (sufentanil), the flow rate, the infusion mode, consumables, and some injection history information (such as pressure information, etc). The same interface can also display some soft controls controlling the syringe pump, such as a fast push control or a pause control. The user can enable the processor to drive for speeding up the injection by contacting the fast push control or inputting a gesture based on the fast push control. The user can enable the processor to stop the operation of the syringe pump drive mechanism by touching the pause control or inputting a gesture based on the pause control. During the injection process, the medicine amount information and/or the injection time information are displayed on the display interface of the injection pump, which greatly facilitates the medical staff, so that the medical staff can make the next work arrangement according to the medicine amount information and/or the injection time information, but not need to pay frequent attention to the injection progress of the injection pump.

In some embodiments, a method for confirming infusion information of a syringe pump is provided, wherein the method is applied to the syringe pump which includes a syringe pump drive mechanism, a syringe clamping mechanism, a syringe propulsion mechanism, a processor, an output interface and a display screen. The method includes:

clamping a syringe by the syringe clamping mechanism;
inputting syringe model information;
displaying medicine amount information;
wherein the medicine amount information changes along with operation of the syringe pump drive mechanism; and when the syringe pump drive mechanism operates, the syringe pump drive mechanism drives the syringe propulsion mechanism to push a piston of the syringe for enabling a relative movement between the body of the syringe and the piston for implementing an operation.

In some embodiments, the syringe pump includes a first sensor and a second sensor. The first sensor is associated with the syringe clamping mechanism. The second sensor is associated with the syringe propulsion mechanism, wherein before displaying the medicine amount information, the method further includes:

> determining a radial length of the syringe according to a feedback signal of the first sensor;
> determining a displacement of the piston relative to the body of the syringe according to a feedback signal of the second sensor;
> determining a length of a remaining medicine amount, according to the displacement of the piston relative to the body of the syringe and the syringe model information; and
> determining the medicine amount information according to the radial length of the syringe and the length of the remaining medicine amount.

In some embodiments, the syringe pump also includes a memory and a second sensor. The memory is operable to store a preset radial length of the syringe corresponding to the syringe model. The second sensor is associated with the syringe propulsion mechanism, wherein before displaying the medicine amount information, the method further includes:

> retrieving a radial length of the syringe stored by the memory;
> determining a displacement of the piston relative to the body of the syringe according to a feedback signal of the second sensor;
> determining a length of a remaining medicine amount, according to the displacement of the piston relative to the body of the syringe and the syringe model information;
> determining the medicine amount information according to the radial length of the syringe and the length of the remaining medicine amount.

In some embodiments, determining a displacement of the piston relative to the body of the syringe according to a feedback signal of the second sensor, includes:

> determining a start position of the piston and a current position of the piston according to the feedback signal of the second sensor; and
> determining the displacement of the piston relative to the body of the syringe according to the start position of the piston and the current position of the piston.

In some embodiments, determining a displacement of the piston relative to the body of the syringe according to a feedback signal of the second sensor, includes:

> determining a start position of the piston according to the feedback signal of the second sensor; and
> determining the displacement of the piston relative to the body of the syringe according to the start position of the piston and a number of revolutions of a motor.

In some embodiments, a method for confirming infusion information of a syringe pump is provided, wherein the method is applied to the syringe pump which includes a syringe pump drive mechanism, a syringe clamping mechanism, a syringe propulsion mechanism, a processor, an output interface and a display screen. The method includes:

> clamping a syringe by the syringe clamping mechanism;
> inputting syringe model information and a flow rate;
> displaying injection time information;
> wherein the injection time information changes along with operation of the syringe pump drive mechanism; and when the syringe pump drive mechanism operates, the syringe pump drive mechanism drives the syringe propulsion mechanism to push a piston of the syringe for enabling a relative movement between the body of the syringe and the piston for implementing an operation.

In some embodiments, the syringe pump includes a first sensor and a second sensor. The first sensor is associated with the syringe clamping mechanism. The second sensor is associated with the syringe propulsion mechanism, wherein before displaying the injection time information, the method further includes:

> determining a radial length of the syringe according to a feedback signal of the first sensor;
> determining a displacement of the piston relative to the body of the syringe according to a feedback signal of the second sensor;
> determining the injection time information according to the radial length of the syringe, the displacement and the flow rate.

In some embodiments, the syringe pump also includes a memory and a second sensor. The memory is operable to store a preset radial length of the syringe corresponding to the syringe model. The second sensor is associated with the syringe propulsion mechanism, wherein before displaying the injection time information, the method further includes:

> retrieving a radial length of the syringe stored by the memory;

determining a displacement of the piston relative to the body of the syringe according to a feedback signal of the second sensor;

determining the injection time information according to the radial length of the syringe, the displacement and the flow rate.

In some embodiments, determining the injection time information according to the radial length of the syringe, the displacement and the flow rate, includes:

determining a length of a remaining medicine amount, according to the displacement of the piston relative to the body of the syringe and the syringe model information;

determining the medicine amount information according to the radial length of the syringe and the length of the remaining medicine amount;

determining the injection time information according to the medicine amount information and the flow rate.

In some embodiments, determining a displacement of the piston relative to the body of the syringe according to a feedback signal of the second sensor, includes:

determining a start position of the piston and a current position of the piston according to the feedback signal of the second sensor; and

determining the displacement of the piston relative to the body of the syringe according to the start position of the piston and the current position of the piston.

In some embodiments, determining a displacement of the piston relative to the body of the syringe according to a feedback signal of the second sensor, includes:

determining a start position of the piston according to the feedback signal of the second sensor; and

determining the displacement of the piston relative to the body of the syringe according to the start position of the piston and a number of revolutions of a motor.

The description of the above method embodiment is consistent with that of each embodiment of the syringe pump, and will not be repeated here.

In some embodiments, a medical device is provided, which is operable to connect with the syringe pump 10 described above through the output interface, wherein the medical device is provided with a display device which is operable to display instruction information which is outputted by the output interface.

The medical device can be a monitor, which is operable to display physiological parameters and personal information of the patient. The medical device can also be an infusion support Dock. The infusion support has multiple notches, which can place multiple infusion pumps or syringe pumps respectively. The infusion pump or syringe pump is physically connected with the infusion support, and is communicated with or charged by a router or a power interface placed on the infusion support. A display is placed on the infusion support and used to display the infusion information or alarm information or personal information of patient for each inserted infusion pump or syringe pump.

A storage medium is provided, which stores executable instructions, wherein when the executable instructions are executed by a processor, steps in the method above can be implemented.

Those skilled in the art can understand that, all or some of the process for implementing the method in the above embodiment, can be completed by instructing related hardware through a computer program. The computer program can be stored in a nonvolatile computer readable storage medium. When the computer program is executed, it can include the processes of the above embodiments. Any reference to the memory, storage, database or other media used in the embodiments provided by the present disclosure may include nonvolatile and/or volatile memory. The nonvolatile memory may include read only memory (ROM), programmable ROM (PROM), electrically programmable ROM (EPROM), electrically erasable programmable ROM (EEPROM), or flash memory. The volatile memory may include random access memory (RAM) or external cache memory. As an illustration rather than limitation, RAM is available in various forms, such as static RAM (SRAM), dynamic RAM (DRAM), synchronous DRAM (SDRAM), dual data rate SDRAM (DDRSDRAM), enhanced SDRAM (ESDRAM), synchronous link (Synchlink) DRAM (SLDRAM), rambus direct RAM (RDRAM), direct memory bus dynamic RAM (DRDRAM), memory bus dynamic RAM (RDRAM), etc.

The technical features of the above embodiments can be combined arbitrarily. In order to make the description concise, not all possible combinations of the technical features in the above embodiments are described. However, as long as there is no contradiction in the combination of these technical features, they should be considered as falling in the scope recorded in this description.

The above embodiments only describe several embodiments of this disclosure, and the description is more specific and detailed, but it cannot be understood as limiting the scope of the present disclosure. It should be noted that for those skilled in the art, several modifications and improvements can be made without departing from the concept of this disclosure, and these modifications and improvements still belong to the protection scope of this disclosure. Therefore, the protection scope of this application shall be subject to the appended claims.

## Claims

1. A syringe pump, **characterized in that**, the syringe pump comprises a syringe pump drive mechanism, a syringe clamping mechanism, a syringe propulsion mechanism, a processor, an output interface and a display screen;

   wherein the syringe clamping mechanism is operable to clamp a body of a syringe, the syringe pump drive mechanism is operable to drive the syringe propulsion mechanism to push a piston of the syringe for enabling a relative movement between the body of the syringe and the piston of the syringe;
   the processor is operable to connect with the display screen through the output interface; and the processor is operable to display medicine amount information on the display screen, after the syringe is clamped by the syringe clamping mechanism and syringe model information is inputted by a user;
   wherein the medicine amount information changes along with operation of the syringe pump drive mechanism.

2. The syringe pump according to claim 1, **characterized in that**, the syringe pump further comprises a first sensor and a second sensor;
   the first sensor is associated with the syringe clamping mechanism, and the processor is operable to determine a radial length of the syringe according to a feedback signal of the first sensor; the second sensor is associated with the syringe propulsion mechanism, and the processor is operable to determine a displacement of the piston relative to the body of the syringe according to a feedback signal of the second sensor; wherein the processor is further operable to determine the medicine amount information according to the radial length of the syringe and the displacement.

3. The syringe pump according to claim 2, **characterized in that**, the syringe pump further comprises a memory which is operable to store an output function of the first sensor, wherein the output function characterizes a functional relationship between the feedback signal of the first sensor and the radial length of the syringe;
   the processor is operable to determine the medicine amount information according to the output function, the radial length of the syringe and the displacement.

4. The syringe pump according to claim 2, **characterized in that**, the first sensor comprises a rotary potentiometer, a Hall sensor, or a magnetoresistive sensor.

5. The syringe pump according to claim 1, **characterized in that**, the syringe pump further comprises a memory and a second sensor;

   the memory is operable to store a preset radial length of the syringe corresponding to the syringe model, the second sensor is associated with the syringe propulsion mechanism, and the processor is operable to determine a displacement of the piston relative to the body of the syringe according to a feedback signal of the second sensor;
   wherein the processor is further operable to retrieve the radial length of the syringe stored in the memory and determine the medicine amount information according to the radial length of the syringe and the displacement.

6. The syringe pump according to claim 2 or 5, **characterized in that**, in order to determine the displacement of the piston relative to the body of the syringe according to the feedback signal of the second sensor, the processor is specifically operable to:

   determine a start position of the piston and a current position of the piston according to the feedback signal of the second sensor, and determine the displacement of the piston relative to the body of the syringe according to the start position of the piston and the current position of the piston

7. The syringe pump according to claim 6, **characterized in that**, the second sensor comprises a slide potentiometer, a film potentiometer, a grating ruler or a capacitive grating ruler.

8. The syringe pump according to claim 2 or 5, **characterized in that**, in order to determine the displacement of the piston relative to the body of the syringe according to the feedback signal of the second sensor, the processor is specifically operable to:

   determine a start position of the piston according to the feedback signal of the second sensor; and determine the displacement of the piston relative to the body of the syringe according to the start position of the piston and a number of revolutions of a motor.

9. The syringe pump according to claim 2 or 5, **characterized in that**, the syringe pump further comprises a memory which is operable to store an output function of the second sensor, wherein the output function characterizes a functional relationship between the feedback signal of the second sensor and the displacement of the piston relative to the body of the syringe;

wherein the processor is operable to determine the medicine amount information according to the output function, the radial length of the syringe and the displacement.

10. A syringe pump, **characterized in that**, the syringe pump comprises a syringe pump drive mechanism, a syringe clamping mechanism, a syringe propulsion mechanism, a processor, an output interface and a display screen;

wherein the syringe clamping mechanism is operable to clamp a body of a syringe, the syringe pump drive mechanism is operable to drive the syringe propulsion mechanism to push a piston of the syringe for enabling a relative movement between the body of the syringe and the piston of the syringe;
the processor is operable to connect with the display screen through the output interface; and the processor is operable to display injection time information on the display screen, after the syringe is clamped by the syringe clamping mechanism, and syringe model information and a flow rate are inputted by a user;
wherein the injection time information changes along with operation of the syringe pump drive mechanism.

11. The syringe pump according to claim 10, **characterized in that**, the syringe pump further comprises a first sensor and a second sensor;
the first sensor is associated with the syringe clamping mechanism, and the processor is operable to determine a radial length of the syringe according to a feedback signal of the first sensor; the second sensor is associated with the syringe propulsion mechanism, and the processor is operable to determine a displacement of the piston relative to the body of the syringe according to a feedback signal of the second sensor; wherein the processor is further operable to determine the injection time information according to the radial length of the syringe, the displacement and the flow rate.

12. The syringe pump according to claim 11, **characterized in that**, the syringe pump further comprises a memory which is operable to store an output function of the first sensor, wherein the output function characterizes a functional relationship between the feedback signal of the first sensor and the radial length of the syringe;
the processor is operable to determine the injection time information according to the output function, the radial length of the syringe, the displacement and the flow rate.

13. The syringe pump according to claim 11, **character-**

**ized in that**, the first sensor comprises a rotary potentiometer, a Hall sensor, or a magnetoresistive sensor.

14. The syringe pump according to claim 10, **characterized in that**, the syringe pump further comprises a memory and a second sensor;

the memory is operable to store a preset radial length of the syringe corresponding to the syringe model, the second sensor is associated with the syringe propulsion mechanism, and the processor is operable to determine a displacement of the piston relative to the body of the syringe according to a feedback signal of the second sensor;
wherein the processor is further operable to retrieve the radial length of the syringe stored in the memory and determine the injection time information according to the radial length of the syringe, the displacement and the flow rate.

15. The syringe pump according to claim 11 or 14, **characterized in that**, in order to determine the displacement of the piston relative to the body of the syringe according to the feedback signal of the second sensor, the processor is specifically operable to:

determine a start position of the piston and a current position of the piston according to the feedback signal of the second sensor, and determine the displacement of the piston relative to the body of the syringe according to the start position of the piston and the current position of the piston.

16. The syringe pump according to claim 15, **characterized in that**, the second sensor comprises a slide potentiometer, a film potentiometer, a grating ruler or a capacitive grating ruler.

17. The syringe pump according to claim 11 or 14, **characterized in that**, in order to determine the displacement of the piston relative to the body of the syringe according to the feedback signal of the second sensor, the processor is specifically operable to: determine a start position of the piston according to the feedback signal of the second sensor; and determine the displacement of the piston relative to the body of the syringe according to the start position of the piston and a number of revolutions of a motor.

18. The syringe pump according to claim 11 or 14, **characterized in that**, the syringe pump further comprises a memory which is operable to store an output function of the second sensor, wherein the output function characterizes a functional relationship between the feedback signal of the second sensor and the displacement of the piston relative to the body of the syringe;

wherein the processor is operable to determine the injection time information according to the output function, the radial length of the syringe and the displacement.

19. A syringe pump, **characterized in that**, the syringe pump comprises a syringe pump drive mechanism, a syringe clamping mechanism, a syringe propulsion mechanism, a processor, an output interface and a display screen;

   wherein the syringe clamping mechanism is operable to clamp a body of a syringe, the syringe pump drive mechanism is operable to drive the syringe propulsion mechanism to push a piston of the syringe for enabling a relative movement between the body of the syringe and the piston of the syringe;
   the processor is operable to connect with the display screen through the output interface; and the processor is operable to display medicine amount information and/or injection time information on the display screen, after the syringe is clamped by the syringe clamping mechanism, and syringe model information and a flow rate are inputted by a user;
   wherein the medicine amount information and/or the injection time information change(s) along with operation of the syringe pump drive mechanism; wherein the medicine amount information or the injection time information is displayed in a first display mode; wherein a ratio between the medicine amount information and a total infusion amount, or a contrast between the injection time information and a total infusion time, is displayed in a second display mode; wherein the first display mode or the second display mode includes text, number or graphics or combination thereof, and the first display mode is different from the second display mode.

20. The syringe pump according to claim 19, **characterized in that**, the graphics include a progress bar for indication; wherein when the medicine amount information or the injection time information is greater than a first preset threshold, a color of the progress bar for indication is a first preset color, when the medicine amount information or the injection time information is less than the first preset threshold but greater than a second preset threshold, the color of the progress bar for indication is a second preset color, and when the medicine amount information or the injection time information is less than the second preset threshold, the color of the progress bar for indication is a third preset color.

21. The syringe pump according to claim 20, **characterized in that**, the processor is operable to determine an infusion end time according to the injection time information and a current time, and to send a reminder signal before a preset time for work shift when a difference between the infusion end time and the preset time for work shift is less than a third preset threshold.

22. A method for confirming infusion information of a syringe pump, **characterized in that**, the method is applied to the syringe pump which comprises a syringe pump drive mechanism, a syringe clamping mechanism, a syringe propulsion mechanism, a processor, an output interface and a display screen, wherein the method comprises:

   clamping a syringe by the syringe clamping mechanism;
   inputting syringe model information;
   displaying medicine amount information;
   wherein the medicine amount information changes along with operation of the syringe pump drive mechanism; and when the syringe pump drive mechanism operates, the syringe pump drive mechanism drives the syringe propulsion mechanism to push a piston of the syringe for enabling a relative movement between a body of the syringe and the piston of the syringe for implementing an operation.

23. The method according to claim 22, **characterized in that**, the syringe pump comprises a first sensor and a second sensor; wherein the first sensor is associated with the syringe clamping mechanism, the second sensor is associated with the syringe propulsion mechanism, wherein before displaying the medicine amount information, the method further comprises:

   determining a radial length of the syringe according to a feedback signal of the first sensor;
   determining a displacement of the piston relative to the body of the syringe according to a feedback signal of the second sensor;
   determining a length of a remaining medicine amount, according to the displacement of the piston relative to the body of the syringe and the syringe model information; and
   determining the medicine amount information according to the radial length of the syringe and the length of the remaining medicine amount.

24. The method according to claim 22, **characterized in that**, the syringe pump further comprises a memory and a second sensor; wherein the memory is operable to store a preset radial length of the syringe corresponding to the syringe model, the second sensor is associated with the syringe propulsion mechanism, wherein before displaying the medicine amount information, the method further comprises:

retrieving a radial length of the syringe stored by the memory;
determining a displacement of the piston relative to the body of the syringe according to a feedback signal of the second sensor;
determining a length of a remaining medicine amount, according to the displacement of the piston relative to the body of the syringe and the syringe model information;
determining the medicine amount information according to the radial length of the syringe and the length of the remaining medicine amount.

25. A method for confirming infusion information of a syringe pump, **characterized in that**, the method is applied to the syringe pump which comprises a syringe pump drive mechanism, a syringe clamping mechanism, a syringe propulsion mechanism, a processor, an output interface and a display screen, wherein the method comprises:

clamping a syringe by the syringe clamping mechanism;
inputting syringe model information and a flow rate;
displaying injection time information;
wherein the injection time information changes along with operation of the syringe pump drive mechanism; and when the syringe pump drive mechanism operates, the syringe pump drive mechanism drives the syringe propulsion mechanism to push a piston of the syringe for enabling a relative movement between a body of the syringe and the piston of the syringe for implementing an operation.

26. The method according to claim 25, **characterized in that**, the syringe pump comprises a first sensor and a second sensor; wherein the first sensor is associated with the syringe clamping mechanism and the second sensor is associated with the syringe propulsion mechanism; wherein before displaying the injection time information, the method further comprises:

determining a radial length of the syringe according to a feedback signal of the first sensor;
determining a displacement of the piston relative to the body of the syringe according to a feedback signal of the second sensor;
determining the injection time information according to the radial length of the syringe, the displacement and the flow rate.

27. The method according to claim 25, **characterized in that**, the syringe pump also comprises a memory and a second sensor; wherein the memory is operable to store a preset radial length of the syringe corresponding to the syringe model, the second sensor is associated with the syringe propulsion mechanism; wherein before displaying the injection time information, the method further comprises:

retrieving a radial length of the syringe stored by the memory;
determining a displacement of the piston relative to the body of the syringe according to a feedback signal of the second sensor;
determining the injection time information according to the radial length of the syringe, the displacement and the flow rate.

28. The method according to claim 26 or 27, **characterized in that**, determining the injection time information according to the radial length of the syringe, the displacement and the flow rate, comprises:

determining a length of a remaining medicine amount, according to the displacement of the piston relative to the body of the syringe and the syringe model information;
determining the medicine amount information according to the radial length of the syringe and the length of the remaining medicine amount;
determining the injection time information according to the medicine amount information and the flow rate.

29. A medical device, **characterized in that**, the medical device is operable to connect with the syringe pump according to any one of claims 1-21 through the output interface, wherein the medical device is provided with a display device which is operable to display instruction information which is outputted by the output interface.

30. A storage medium, **characterized in that**, the storage medium stores executable instructions, wherein when the executable instructions are executed by a processor, the method according to any one of claims 22-28 is implemented.

100

Peripheral device interface 152

Processor/ controller 150

102

110

Power supply system 106

Power drive circuit 130

RF circuit 120

110

152

Bubble sensor 134

External interface 122

Pressure sensor 136

Audio circuit 124

154

Temperature sensor 138

Monitoring circuit 126

Protection circuit 128

104

Operation system 170

Communication module 172

Touch module 174

Tactile feedback module 176

Movement module 178

Position module 180

Graphics module 182

Text input module 190

Device/overall internal state 192

Application 194

I/O system 108

Display system 160

Display controller 140

Other input/ control device 162

Other input controller 142

Position sensor 164

Position sensor controller 144

Displacement sensor 166

Proximity sensor controller 146

168

Light controller 148

Infusion mode setting 194-1

Blocking pressure level setting 194-2

Medicine setting 194-4

Volume setting 194-5

Brightness setting 194-6

Online setting 195-7

Dock setting 195-8

Temperature setting 195-9

......

FIG.1

10

Syringe pump  12

| Processor | 12 |

13

| Output interface | 13 |

| Syringe | 11 |

14

| Display screen | 14 |

FIG.2

10

20

22

| Processor | 12 |

| Syringe pump driving mechanism | 20 |

23

24

21

FIG.3

10

22

12

Processor

20

Syringe pump
driving
mechanism 20

221

222

223

31

225

23

224

21

24

30

FIG.4

FIG.5

FIG.6

FIG.7

Remaining amount    40 ml

Infused amount    10 ml

⇩

803

Remaining amount    15 ml

Infused amount    35 ml

⇩

805

Remaining amount    5 ml

Infused amount    45 ml

FIG.8A

Remaining time 2h 30min

807

⇩

Remaining time 1h 30min

809

⇩

Remaining time  30min

811

FIG.8B

813

⇩

815

⇩

Remaining amount 35ml

817        819

FIG.8C

821

⇓

823

⇓

Remaining time 2h 30min

825

827

FIG.8D

Infusion is about to be completed!
Please dispense medicines in time

×

830

FIG.9A

30 minutes to go! Please prepare medicines for next shift in advance

Remaining time     30min

**FIG.9B**

| | | | |
|---|---|---|---|
| | | Time Amount/Hour Total amount | |
| Infusing «««««« | Remaining amount 40 ml | 09:30    3    3 | Push quickly |
| Sufentanil 15.00 ml/h | Infused amount 10 ml | 10:30    4    7 | |
| | | 11:30    10    17 | |
| Rate mode | Braun-away from light | Remaining time 03 h 20 min | Blocking pressure | Pause |

**FIG.10**

## INTERNATIONAL SEARCH REPORT

| | International application No. |
|---|---|
| | **PCT/CN2019/107793** |

### A. CLASSIFICATION OF SUBJECT MATTER

A61M 5/20(2006.01)i; A61M 5/142(2006.01)i

According to International Patent Classification (IPC) or to both national classification and IPC

### B. FIELDS SEARCHED

Minimum documentation searched (classification system followed by classification symbols)

A61M

Documentation searched other than minimum documentation to the extent that such documents are included in the fields searched

Electronic data base consulted during the international search (name of data base and, where practicable, search terms used)

CNKI, CNPAT, WPI, EPODOC: 深圳迈瑞, 杨雷, 张鹏, 邹小玲, 注射, 输注, 输液, 泵, 注射器, 驱动, 传动, 夹持, 卡紧, 推进, 推动, 活塞, 处理器, 控制器, 显示, 传感器, 剩余, 药量, 时间, 半径, 直径, 位移, 长度, injector, syringe, pump, driv+, hold +, retain+, clamp+, grip+, impel+, propel+, push+, piston, plunger, processor, controller, display, sensor, remain+, time, dose, radius, diameter, motion, displacement, length

### C. DOCUMENTS CONSIDERED TO BE RELEVANT

| Category* | Citation of document, with indication, where appropriate, of the relevant passages | Relevant to claim No. |
|---|---|---|
| X | CN 107349492 A (LIAOCHENG FEIRAN SHIKAI TECHNOLOGY CO., LTD.) 17 November 2017 (2017-11-17) description paragraphs [0028], [0050], [0053], [0056]-[0060], [0065], [0072], [0084]-[0086], [0089], [0093]-[0097], figures 1-7 | 1, 5-10, 14-22, 24-25, 27-30 |
| Y | CN 107349492 A (LIAOCHENG FEIRAN SHIKAI TECHNOLOGY CO., LTD.) 17 November 2017 (2017-11-17) description paragraphs [0028], [0050], [0053], [0056]-[0060], [0065], [0072], [0084]-[0086], [0089], [0093]-[0097], figures 1-7 | 2-4, 11-13, 23, 26 |
| Y | CN 109718409 A (SWS HEMODIALYSIS CARE CO., LTD.) 07 May 2019 (2019-05-07) description, paragraphs [0060], [0063] and [0064], and figures 1 and 3-4 | 2-4, 11-13, 23, 26 |
| X | CN 204395143 U (YUNXI PEOPLE'S HOSPITAL) 17 June 2015 (2015-06-17) description, paragraphs [0016]-[0022], and figure 1 | 1, 10, 19-22, 25, 29-30 |
| A | CN 2259182 Y (SUZHOU MEDICAL COLLEGE) 13 August 1997 (1997-08-13) entire document | 1-30 |

☑ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **04 June 2020** | **23 June 2020** |

| Name and mailing address of the ISA/CN | Authorized officer |
|---|---|
| **China National Intellectual Property Administration (ISA/CN)** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2019/107793**

**C.    DOCUMENTS CONSIDERED TO BE RELEVANT**

| Category* | Citation of document, with indication, where appropriate, of the relevant passages | Relevant to claim No. |
|---|---|---|
| A | CN 101839702 A (SINO MEDICAL-DEVICE TECHNOLOGY CO., LTD.) 22 September 2010 (2010-09-22) entire document | 1-30 |
| A | WO 2019175836 A1 (ONEFUSION AG.) 19 September 2019 (2019-09-19) entire document | 1-30 |

Form PCT/ISA/210 (second sheet) (January 2015)

**INTERNATIONAL SEARCH REPORT**
Information on patent family members

International application No.

**PCT/CN2019/107793**

| Patent document cited in search report | | | Publication date (day/month/year) | Patent family member(s) | | | Publication date (day/month/year) |
|---|---|---|---|---|---|---|---|
| CN | 107349492 | A | 17 November 2017 | None | | | |
| CN | 109718409 | A | 07 May 2019 | None | | | |
| CN | 204395143 | U | 17 June 2015 | None | | | |
| CN | 2259182 | Y | 13 August 1997 | None | | | |
| CN | 101839702 | A | 22 September 2010 | None | | | |
| WO | 2019175836 | A1 | 19 September 2019 | GB | 2571954 | A | 18 September 2019 |
| | | | | GB | 201804043 | D0 | 25 April 2018 |

Form PCT/ISA/210 (patent family annex) (January 2015)